# EUROPÄISCHE PATENTSCHRIFT

(11) **EP 1 139 987 B1**
(45) Veröffentlichungstag und Bekanntmachung des Hinweises auf die Patenterteilung: **21.07.2004**
(21) Anmeldenummer: 99963525.3
(22) Anmeldetag: 14.12.1999
(51) Int. Cl.: A61K 7/13

(54) **FÄRBEMITTEL MIT MANGANKATIONEN**
COLORING AGENTS COMPRISING MANGANESE CATIONS
COLORANTS CONTENANT DES CATIONS DE MANGANESE

(30) Priorität: 23.12.1998 DE 19859682
(43) Veröffentlichungstag der Anmeldung: 10.10.2001
(73) Patentinhaber: Henkel Kommanditgesellschaft auf Aktien, 40589 Düsseldorf-Holthausen (DE)
(72) Erfinder: KUHM, Peter, D-40724 Hilden (DE); EWALD, Gertrud, D-42655 Solingen (DE); HÖFFKES, Horst, D-40595 Düsseldorf (DE)
(86) Internationale Anmeldenummer: PCT/EP1999/009902
(87) Internationale Veröffentlichungsnummer: WO 2000/038624

(56) Entgegenhaltungen:
- EP-A- 0 697 210
- EP-A- 0 749 748
- EP-A- 0 850 635
- US-A- 4 776 856

## Beschreibung

Die vorliegende Erfindung betrifft Mittel zum Färben keratinischer Fasern, die Mangankationen in hohen Oxidationsstufen als katalytisch wirksame Aktivatoren enthalten sowie deren Verwendung.

Für das Färben von keratinhaltigen Fasern, z.B. Wolle, Pelzen und insbesondere menschlichen Haaren, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung.

Direktziehende Farbstoffe werden unter schonenden Bedingungen appliziert. Ihr Nachteil liegt jedoch darin, daß die Färbungen häufig nur über unzureichende Echtheitseigenschaften verfügen. Mit Oxidationsfarbstoffen lassen sich zwar intensive Färbungen mit guten Echtheitseigenschaften erzielen, die Entwicklung der Farbe geschieht jedoch in der Regel unter dem Einfluß von H₂O₂ oder H₂O₂-Addukten, was Schädigungen der Faser zur Folge haben kann.

Auch Oxidationsfärbemittel mit Luftsauerstoff als sehr mildem Oxidationsmittel wurden bereits vorgeschlagen; im allgemeinen laufen die Oxidationen mit Luftsauerstoff jedoch nicht vollständig ab. Insbesondere in Oxidationshaarfärbemitteln, die in der Regel in einem cremeartigen kosmetischen Träger appliziert werden, findet keine zufriedenstellende Diffusion des Luftsauerstoff zu den Farbstoffvorprodukten statt. Weiterhin wurde, beispielsweise in der EP-B1-0 548 620, vorgeschlagen, den Gehalt an Wasserstoffperoxid in den Oxidationsfärbemitteln auf 1 Gew.-% und weniger zu begrenzen, dem Mittel aber gleichzeitig zur Aktivierung spezielle Enzyme, Peroxidasen, zuzufügen. Diese Ansätze haben aber bisher nicht vollständig überzeugt.

Es besteht daher nach wie vor die Aufgabe, Oxidationsfärbemittel zu entwickeln, die aufgrund ihres geringeren Gehaltes an Oxidationsmitteln schonendere Färbungeverfahren ermöglichen, ohne das Färbeergebnis negativ zu beeinflussen. Besonders vorteilhaft wäre die Möglichkeit eines vollständigen Verzichts auf zusätzliche Oxidationsmittel bei gleichzeitig hoher Färbeleistung der Mittel. Weiterhin bestand die Aufgabe, Färbemittel zu entwickeln, die bei Anwendung auf dem Haar zu natürlichen Färbungen führen.

Es wurde nunmehr überraschenderweise gefunden, daß durch den Einsatz von Mangankationen in hohen Oxidationsstufen als Oxidationskatalysatoren in Haarfärbemitteln auf der Basis bestimmter Entwicklerkomponenten
- die Menge des Oxidationsmittels stark reduziert werden kann oder
- die Ausfärbung alleine mit Luftsauerstoff als Oxidationsmittel erfolgen kann.
   Der Einsatz von Mangankationen in oxidativen Haarfärbemitteln ist prinzipiell bekannt. In der europäischen Patentanmeldung EP-A1-749 748 werden beispielsweise Haarfärbemittel auf Basis mindestens eines ortho-Diaminopyrazols beschrieben, die mindestens ein Mangansalz enthalten. In einer Variante erfolgt die Ausfärbung mit Luft beziehungsweise Luftsauerstoff als einzigem Oxidationsmittel. Dieser Schrift sind aber keinerlei Hinweise zu entnehmen, daß auch Ausfärbungen mit anderen Entwicklersubstanzen auf diese Weise erzielt werden können.
   Ein erster Gegenstand der vorliegenden Erfindung sind daher Mittel zur Färbung keratinischer Fasern, die
- mindestens eine Entwicklerkomponente, ausgewählt aus einer Gruppe, gebildet von p-Phenylendiamin, p-Toluylendiamin, p-Aminophenol, 1-(2'-Hydroxyethyl)-2,5-diaminobenzol, N,N-Bis(2-hydroxyethyl)-p-phenylendiamin, 4-Amino-3-methylphenol, 2-Aminomethyl-4-aminophenol, 4-Amino-2-((diethylamino)-methyl)-phenol, Bis-(2-hydroxy-5-aminophenyl)-methan, 2,4,5,6-Tetraaminopyrimidin, 2-Hydroxy-4,5,6-triaminopyrimidin und 4-Hydroxy-2,5,6-triaminopyrimidin, sowie
- Mangankationen in Oxidationsstufen größer als 2 in Form ihrer in der Anwendungszubereitung löslichen Salze als auch ihrer Komplexe enthalten.

Besonders bevorzugte Entwicklerkomponenten sind p-Phenylendiamin, p-Toluylendiamin, p-Aminophenol, 1-(2'-Hydroxyethyl)-2,5-diaminobenzol, N,N-Bis(2-hydroxyethyl)-p-phenylendiamin, 4-Amino-3-methylphenol, 2-Aminomethyl-4-aminophenol, 2,4,5,6-Tetraaminopyrimidin, 2-Hydroxy-4,5,6-triaminopyrimidin und 4-Hydroxy-2,5,6-triaminopyrimidin.

Besonders bevorzugt sind Mangankationen der Oxidationsstufen 3, 4 und/oder 5.

Ganz besonders bevorzugt sind Mangankationen der Oxidationsstufe 3.

Im Sinne der vorliegenden Erfindung können die Mangankationen sowohl in Form ihrer in der Anwendungszubereitung löslichen Salze als auch ihrer Komplexe vorliegen.

Falls die Mangankationen in Form ihrer Salze eingesetzt werden, kommen prinzipiell alle physiologisch verträglichen Anionen als Gegenionen in Frage. Erfindungsgemäß besonders bevorzugt sind dabei Nitrat, Hydroxid, Chlorat, Sulfat, Carbonat, Perchlorat, komplexe Anionen wie Hexafluorophosphat, Tetrafluoroborat, die Halogenide wie Chlorid oder die Anionen von Carbonsäuren wie Formiat, Acetat, Fluoracetat, Benzoat oder Citrat.

Als Liganden in den erfindungsgemäß einsetzbaren Mangankomplexen kommen übliche Substanzen sowohl anorganischer als auch organischer Natur in Frage. Zu den organischen Liganden in derartigen Komplexen gehören neben Carboxylaten insbesondere Verbindungen mit primären, sekundären und/oder tertiären Amin- und/oder Alkohol-Funktionen, wie Pyridin, Pyridazin, Pyrimidin, Pyrazin, Imidazol, Pyrazol, Triazol, 2,2'-Bispyridyl-amin. Tris-(2-pyridylmethyl)amin, 1,4,7-Triazacyclononan, 1,4,7-Trimethyl-1,4,7-triazacyclononan, 1,5,9-Trimethyl-1,5,9-triazacyclododecan, (Bis-((1-methylimidazol-2-yl)-methyl))-(2-pyridylmethyl)-amin, N,N'-(Bis-(1-methylimidazol-2-yl)-methyl)-ethylendiamin, N-Bis-(2-benzimidazolylmethyl)-aminoethanol, 2,6-Bis-(bis-(2-benzimidazolylmethyl)aminomethyl)-4-methylphenol, N,N,N',N'-Teirakis-(2-benzimidazolylmethyl)-2-hydroxy-1,3-diaminopropan, 2,6-Bis-(bis-(2-pyridylmethyl)aminomethyl)-4-methylphenol, 1,3-Bis-(bis-(2-benzimidazolylmethyl)aminomethyl)-benzol, Sorbitol, Mannitol, Erythritol, Adonitol, Inositol, Lactose, und gegebenenfalls substituierte Salene, Porphine und Porphyrine. Zu den anorganischen Neutralliganden gehören insbesondere Ammoniak und Wasser.

Falls nicht sämtliche Koordinationsstellen des Mangankations durch Neutralliganden besetzt sind, enthält ein gemäß der Erfindung zu verwendender Komplex weitere, vorzugsweise anionische und unter diesen insbesondere ein- oder zweizähnige Liganden. Zu diesen gehören insbesondere die Halogenide wie Fluorid, Chlorid, Bromid und Iodid, und die (NO₂)⁻-Gruppe. Unter einer (NO₂)⁻-Gruppe soll im vorliegenden Fall ein Nitro-Ligand, der über das Stickstoffatom an das Mangankation gebunden ist, oder ein Nitrito-Ligand, der über ein Sauerstoffatom an das Mangankation gebunden ist, verstanden werden. Die (NO₂)⁻-Gruppe kann an ein Mangankation auch chelatbildend gebunden sein oder sie kann zwei Mangankationen asymmetrisch oder µ'-O-verbrücken. Außer den genannten Liganden können die im Aktivatorsystem gemäß der Erfindung zu verwendenden Mangankomplexe noch weitere, in der Regel einfacher aufgebaute Liganden, insbesondere ein- oder mehrwertige Anionliganden, tragen. In Frage kommen beispielsweise Nitrat, Actetat, Trifluoracetat, Formiat, Carbonat, Citrat, Perchlorat sowie komplexe Anionen wie Hexafluorophosphat. Die Anionliganden sollen für den Ladungsausgleich zwischen Mangankation und dem Ligandensystem sorgen. Auch die Anwesenheit von Oxo-Liganden, Peroxo-Liganden und Imino-Liganden ist möglich. Insbesondere derartige Liganden können auch verbrückend wirken, so daß mehrkernige Komplexe entstehen. Auch der Einsatz zweikerniger oder mehrkerniger Komplexe, in denen die Mangankationen unterschiedliche Oxidationszahlen aufweisen, ist möglich.

Falls Anionliganden fehlen oder die Anwesenheit von Anionliganden nicht zum Ladungsausgleich im Komplex führt, können in den gemäß der Erfindung zu verwendenden Mangankomplex-Verbindungen anionische Gegenionen anwesend sein, die den kationischen Mangankomplex neutralisieren. Zu diesen anionischen Gegenionen gehören insbesondere Nitrat, Hydroxid, Hexafluorophosphat, Sulfat, Chlorat, Perchlorat, die Halogenide wie Chlorid oder die Anionen von Carbonsäuren wie Formiat, Acetat, Benzoat oder Citrat.

Ganz besonders bevorzugt ist der Einsatz des Dihydrats des Mangan(III)triacetats in den erfindungsgemäßen Haarfärbemitteln.

Die erfindungsgemäßen Verbindungen können sowohl in löslicher Form eingesetzt werden, als auch, wenn es sich um kationische Verbindungen handelt, auf einem Träger fixiert sein.

Erfindungsgemäß einsetzbare Trägermaterialien sind beispielsweise Zeolithe, Schichtsilicate oder Sol/Gel-Systeme.

Zeolithe sind Alumosilicate aus dreidimensional vernetzten Aluminat- und Silicat-Tetraederbausteinen. Alle Zeolithe weisen ein ein- oder mehrdimensionales Kanal- und Porensystem auf. Zu den bevorzugten Trägermaterialien gehören die Zeolithe vom Typ A, K, L, P-L, O, T, X, Y und Ω sowie deren Mischungen. Die erfindungsgemäßen Manganverbindungen können sowohl auf der Oberfläche des Zeolithen vorliegen als auch sich innerhalb der Poren befinden beziehungsweise innerhalb der Poren eingeschlossen sein. Der Ladungsausgleich kann dabei auch zumindest anteilsweise durch den Zeolithen erfolgen.

Erfindungsgemäß einsetzbare Schichtsilicate sind ebenfalls Alumosilicate, die aber nicht die zeolith-typische Porenstruktur aufweisen. Schichtsilicate bilden im suspendierten Zustand vielmehr plättchenförmige Gebilde, auf deren Oberfläche sich die erfindungsgemäßen Manganverbindungen anlagern können. Beispiele für erfindungsgemäße Schichtsilicate ist neben Bentonit, als üblichem Gemisch von Montmorillonit und Kaolinit, weiterhin das unter dem Handelsnamen Dehydril® HT von der Firma Henkel vertriebene Produkt aus äußerst quellfähigem Hectorit.

Erfindungsgemäße Sol/Gel-Systeme sind amorphe, glasartige Substanzen, die aus interpenetrierenden anorganischen (silicatischen) und gegebenenfalls organischen (polymeranalogen) Netzwerken aufgebaut sind. Sol/Gel-Systeme können sowohl auf Basis von Siliciumdioxid als auch auf der Basis von Titandioxid synthetisiert werden.

Zur Nuancierung der erzielbaren Farbtöne können die erfindungsgemäßen Mittel weiterhin noch eine oder mehrere Kupplerkomponenten sowie weitere Entwicklerkomponenten enthalten. Kupplersubstanzen sind häufig aromatische oder heterocyclische Ringsysteme, die zwei reaktive Gruppen in meta-Stellung aufweisen. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind 1-Naphthol, Pyrogallol, 1,5-, 2,7-und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 2,6-Bis-(2-hydroxyethylamino)-toluol, 2,4-Diaminophenoxyethanol, 1-Methoxy-2-amino-4-(2-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 2-Hydroxy-4-aminophenoxyethanol, 2-Methyl-5-(2-hydroxyethylamino)-phenol und 2.6-Dihydroxy-3,4-dimethylpyridin.

Besonders bevorzugte Kuppler-Komponenten sind 1-Naphthol, 1,5-, 2,7- und 1,7-Dihydroxynaphthalin, 5-Amino-2-methylphenol, Resorcin, 3-Aminophenol, 4-Chlorresorcin, 2-Chlor-6-methyl-3-aminophenol, 2-Methylresorcin, 5-Methylresorcin, 2,5-Dimethylresorcin sowie 2,6-Dihydroxy-3,4-diaminopyridin.

Erfindungsgemäß bevorzugte weitere Entwicklerkomponenten sind o-Aminophenol, 2-(2,5-Diaminophenoxy)-ethanol, 1-Phenyl-3-carboxyamido-4-amino-pyrazolon-5, 2,4-Dihydroxy-5,6-diaminopyrimidin, 2-Dimethylamino-4,5,6-triaminopyrimidin, 2-Hydroxymethylamino-4-amino-phenol, 4,4'-Diaminodiphenylamin, 4-Amino-3-fluorphenol, 2-Hydroxymethyl-4-aminophenol, 1,4-Bis-(4-aminophenyl)-diazacycloheptan, 1,3-Bis(N(2-hydroxyethyl)-N(4-aminophenyl-amino))-2-propanol, 4-Amino-2-(2-hydroxyethoxy)-phenol sowie 4,5-Diaminopyrazol-Derivate nach EP 0 740 931 bzw. WO 94/08970 wie z.B. 4,5-Diamino-1-(2'-hydroxyethyl)-pyrazol.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Üblicherweise werden Entwicklerkomponenten und Kupplerkomponenten in etwa gleichen molaren Mengen zueinander eingesetzt. Wenn sich auch der äquimolare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten bevorzugt in einem Mol-Verhältnis von 1 : 0,5 bis 1 : 2 im Färbemittel enthalten sein können. Die Gesamtmenge an Oxidationsfarbstoffvorprodukten liegt in der Regel bei höchstens 20 Gew.-%, bezogen auf das gesamte Mittel.

In einer bevorzugten Ausführungsform enthalten die erfindungsgemäßen Haarfärbemittel zur weiteren Modifizierung der Farbnuancen neben den Farbstoffvorprodukten zusätzlich übliche direktziehende Farbstoffe. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Es ist nicht erforderlich, daß die Farbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbemitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Färbungen von besonderer Farbtiefe können erreicht werden, wenn die Mittel neben den Farbstoffen und/oder Farbstoffvorprodukten zusätzlich noch ein Öl des Wiesenschaumkrauts (INCI-Bezeichnung: Meadowfoam Seed Oil) enthalten.

Zur Herstellung der erfindungsgemäßen Färbemittel werden die Farbstoffvorprodukte in einen geeigneten wäßrigen, alkoholischen oder wäßrig-alkoholischen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen. die für die Anwendung auf dem Haar geeignet sind.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol. zu verstehen.

Die erfindungsgemäßen Färbemittel können weiterhin alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische; ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Färbemittel alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel K-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylen glykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungs produkte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure. Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂- bis C₂₂-Fettsäuremono- und -diester von Anlagerungsprodukten von 1 bis 30 Mol Ethylenoxid an Glycerin,
- C₈- bis C₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethyl-ammoniumglycinate, beispielsweise das Kokosacylaminopropyl-dimethyl-ammonium-glycinat, und 2-Alkyl-3-carboxymethyl-3-hydroxyethyl-imidazoline mit jeweils 8 bis 18 C-Atomen in der Alkyl- oder Acylgruppe sowie das Kokosacyl-aminoethyl-hydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈- bis C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂bis C₁₈-Acylsarcosin.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldi-methylammoniumchloride und Trialkylmethylammoniumchloride, z. B. Cetyltrimethyl-ammoniumchlorid, Stearyltrimethylammoniumchlorid, Distearyldimethylammonium-chlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Corning 929 Emulsion (enthaltend ein hydroxyl-amino-modifiziertes Silikon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyl-dialkoyloxyalkyl-ammonium-methosulfate sowie die unter dem Warenzeichen Dehyquart® vertriebenen Produkte.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Je nach Art des Mittels und des Tensidtyps sind die Tenside in den erfindungsgemäßen Mitteln üblicherweise in Mengen von insgesamt 0,5 bis 30 Gew.-%, bezogen auf das gesamte Mittel, enthalten.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, synthetischen oligomeren Alkenen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quatemisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen.

Als konditionierende Wirkstoffe weiterhin geeignet sind Silikonöle, insbesondere Dialkylund Alkylarylsiloxane, wie beispielsweise Dimethylpolysiloxan und Methylphenylpolysiloxan. sowie deren alkoxylierte und quatemierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Coming; ein stabilisiertes Trimethylsilylamodimethicon), Dow Corning® 929 Emulsion (enthaltend ein hydroxyl-amino-modifiziertes Silikon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle sowie pflanzliche Öle wie Jojobaöl, Sonnenblumenöl, Orangenöl, Mandelöl, Weizenkeimöl und Pfirsichkemöl.

Gleichfalls geeignete haarkonditionierende Verbindungen sind Phospholipide, beispielsweise Sojalecithin, Ei-Lecithin und Kephaline.

Schließlich enthalten die erfindungsgemäßen Färbemittel bevorzugt noch einen Fettstoff.

Bevorzugte Fettstoffe sind lineare und verzweigte, gesättigte und ungesättigte Fettalkohole oder natürliche Fettalkoholgemische mit 8 bis 22 Kohlenstoffatomen in der Alkylkette wie beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Palmitylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole sowie Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden und somit ein Gemisch von unterschiedlichen Fettalkoholen darstellen. Die Fettalkohole werden üblicherweise in Mengen von 0,01 bis 15 Gew.-%, bevorzugt von 0,1 bis 10 Gew.-% und besonders bevorzugt von 0,3 bis 6 Gew.-%, bezogen auf die gesamte Zubereitung, eingesetzt.

Ebenfalls als Fettstoffe eingesetzt werden können Monoester der Fettsäuren mit Alkoholen mit 6 bis 24 C-Atomen sowie Triglyceride natürlichen Ursprungs.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.-Butylacrylamid-Terpolymere,
- symmetrische und unsymmetrische, lineare und verzweigte Dialkylether mit insgesamt zwischen 12 bis 36 C-Atomen, insbesondere 12 bis 24 C-Atomen, beispielsweise Din-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert-butylether, Di-iso-pentylether, Di-3-ethyldecylether, tert.-Butyl-n-octylether, iso-Pentyl-n-octylether und 2-Methyl-pentyl-n-octylether,
- Antischuppenwirkstoffe wie Piroctone Olamine, Zink Omadine und Climbazol,
- tierische und pflanzliche Proteinhydrolysate, insbesondere Elastin-, Kollagen-, Keratin-, Milcheiweiß-, Sojaprotein-, Mandelprotein- und Weizenproteinhydrolysate, sowie deren Fettsäurekondensationsprodukte und quaternierte Derivate,
- Vitamine und Vitaminvorstufen wie Panthenol, dessen Derivate und Biotin,
- Pflanzen- und Honigextrakte, wie insbesondere Extrakte aus Eichenrinden, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel,
- Weitere Wirkstoffe wie Ceramide, Allantoin, Pyrrolidoncarbonsäuren, und Bisabolol,
- Lichtschutzmittel,
- Entschäumer wie Silikone,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Alkalisierungsmittel wie beispielsweise Ammoniak, Monoethanolamin, 2-Amino-2-methylpropanol und 2-Amino-2-methyl-propandiol-1,3
- weitere Substanzen zur Einstellung des pH-Wertes,
- Cholesterin,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Konsistenzgeber wie Zuckerester, Polyolester oder Polyolalkylether,
- Strukturanten wie Maleinsäure, Mono-, Di- und Oligosaccharide,
- Fette und Wachse, wie Walrat, Bienenwachs, Montanwachs und Paraffine,
- Fettsäurealkanolamide,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Komplexbildner wie EDTA, NTA, β-Alanindiessigsäure und Phosphonsäuren,
- Treibmittel wie Propan-Butan-Gemische, N₂O, Dimethylether, CO₂, N₂ und Luft

Bezüglich weiterer Bestandteile sowie Mengenbereiche für die einzelnen Inhaltsstoffe wird auf die dem Fachmann bekannten Handbücher, z.B. K. Schrader, Grundlagen und Rezepturen der Kosmetika, 2. Auflage, Hüthig Buch Verlag, Heidelberg, 1989, verwiesen.

Die oxidative Entwicklung der Färbung kann grundsätzlich allein mit Luftsauerstoff erfolgen. Es ist jedoch nicht ausgeschlossen ein chemisches Oxidationsmittel einzusetzen, besonders dann, wenn neben der Färbung ein Aufhelleffekt am menschlichen Haar gewünscht ist. Als Oxidationsmittel kommen insbesondere Wasserstoffperoxid oder dessen Anlagerungsprodukte an Harnstoff, Melamin oder Natriumborat in Frage. Weiterhin ist es möglich, die Oxidation mit Hilfe von Enzymen durchzuführen. Dabei können die Enzyme sowohl zur Erzeugung von oxidierenden Perverbindungen eingesetzt werden, als auch zu Verstärkung der Wirkung einer geringen Menge vorhandener Oxidationsmittel. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Die Manganverbindungen können erfindungsgemäß sowohl in einer gemeinsamen Zubereitung mit den Farbstoffvorprodukten als auch separat konfektioniert sein.

In einer bevorzugten Ausfuhrungsform der erfindungsgemäßen Lehre werden die separat konfektionierten Manganverbindungen, insbesondere wenn sie auf einem Träger aufgebracht sind, in einem geeigneten Lösemittel, beispielsweise in Wasser, Ethanol oder Aceton, gelöst beziehungsweise suspendiert und unmittelbar vor dem Färben der Haare mit der Zubereitung, enthaltend die Farbstoffvorprodukte, vermischt. Das dabei entstehende gebrauchsfertige Haarfärbepräparat sollte bevorzugt einen pH-Wert im Bereich von 6 bis 10 aufweisen. Besonders bevorzugt ist ein pH-Wert von 6,5 bis 8. Die Anwendungstemperaturen können in einem Bereich zwischen 15 und 40°C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann nach einer Einwirkzeit der Anwendungszubereitung von 20 bis 30 Minuten - gegebenenfalls nach einer Zwischenspülung - eine Oxidationskomponente aufgebracht werden. Nach einer weiteren Einwirkdauer von 10 bis 20 Minuten wird dann gespült und gewünschtenfalls nachshampooniert.

Ein zweiter Gegenstand der vorliegenden Erfindung ist die Verwendung der obengenannten Mittel zur Färbung keratinischer Fasern.

Durch die erfindungsgemäße Verwendung der Manganverbindungen ist es möglich, Haarfärbemittel zu formulieren, die mit Luftsauerstoff als einzigem Oxidationsmittel Haarfärbungen ergeben, die denen entsprechen, die unter Verwendung einer 1 - 3 Gew.-% Wasserstoffperoxidkonzentration erhalten werden.

Die folgenden Beispiele sollen den Gegenstand der vorliegenden Erfindung näher erläutern.

### Beispiele

Es wurden 0,4mmol (50mg) 2-Methylresorcin und 0,4mmol (95 mg) 2,4,5,6-Tetraaminopyrimidinsulfat in 10ml Wasser gelöst und der pH-Wert mit einer wäßrigen Ammoniaklösung auf 9 eingestellt. Anschließend wurden 10mg Mangan(III)acetat-dihydrat in 3ml Wasser gelöst und unmittelbar vor der Anwendung auf dem Haar zu der Farbstoffvorproduktlösung gegeben.

Eine Haarsträhne (Kerling naturweiß) wurde für eine Minute in die Lösung getaucht und anschließend 30 Minuten an der Luft liegen gelassen, bevor sie mit einem Fön getrocknet wurde.

Die Strähne wies eine intensive rote Färbung auf, die nicht von einer Färbung abweicht, die mit einer Zubereitung mit 1 -3 Gew.-% Wasserstoffperoxid erhalten wurde.

## Patentansprüche

1. Mittel zum Färben keratinischer Fasern, insbesondere menschlicher Haare, **dadurch gekennzeichnet, daß** es
• mindestens eine Entwicklerkomponente, ausgewählt aus einer Gruppe, gebildet von p-Phenylendiamin, p-Toluylendiamin, p-Aminophenol, 1-(2'-Hydroxyethyl)-2,5-diaminobenzol, N,N-Bis(2-hydroxyethyl)-p-phenylendiamin, 4-Amino-3-methylphenol, 2-Aminomethyl-4-aminophenol, 4-Amino-2-((diethylamino)-methyl)-phenol, Bis-(2-hydroxy-5-aminophenyl)-methan, 2,4,5,6-Tetraaminopyrimidin, 2-Hydroxy-4,5,6-triaminopyrimidin und 4-Hydroxy-2,5,6-triaminopytimidin, sowie
• Mangankationen in Oxidationsstufen größer als 2 in Form ihrer in der Anwendungszubereitung löslichen Salze und/oder ihrer Komplexe
enthält.

2. Mittel nach Anspruch 1, **dadurch gekennzeichnet, daß** die Entwicklerkomponente, ausgewählt ist aus einer Gruppe, die gebildet wird von p-Phenylendiamin, p-Toluylendiamin, p-Aminophenol, 1-(2'-Hydroxyethyl)-2,5-diaminobenzol, N,N-Bis(2-hydroxyethyl)-p-phenylendiamin, 4-Amino-3-methylphenol, 2-Aminomethyl-4-aminophenol, 2,4,5,6-Tetraaminopyrimidin, 2-Hydroxy-4,5,6-triaminopyrimidin und 4-Hydroxy-2,5,6-triaminopyrimidin.

3. Mittel nach Anspruch 1 oder 2, **dadurch gekennzeichnet, daß** die Mangankationen in den Oxidationsstufen 3, 4 und/oder 5 vorliegen.

4. Mittel nach einem der Ansprüche 1 bis 3, **dadurch gekennzeichnet, daß** die Mangankationen in Form des Dihydrats des Mangan(III)triacetats eingesetzt werden.

5. Mittel nach einem der Ansprüche 1 bis 4, **dadurch gekennzeichnet, daß** die Mangankationen auf einem Träger fixiert sind.

6. Mittel nach einem der Ansprüche 1 bis 5, **dadurch gekennzeichnet, daß** es mindestens eine Kupplerkomponente enthält.

7. Mittel nach einem der Ansprüche 1 bis 6, **dadurch gekennzeichnet, daß** es mindestens einen direktziehenden Farbstoff enthält.

8. Mittel nach einem der Ansprüche 1 bis 7, **dadurch gekennzeichnet, daß** es frei von Wasserstoffperoxid ist.

9. Verwendung eines der Mittel der Ansprüche 1 bis 8 zur oxidativen Färbung keratinischer Fasern.

## Claims

1. A preparation for colouring keratinous fibres, more particularly human hair, **characterized in that** it contains
• at least one primary intermediate selected from a group consisting of p-phenylenediamine, p-toluylenediamine, p-aminophenol, 1-(2'-hydroxyethyl)-2,5-diaminobenzene, N,N-bis-(2-hydroxyethyl)-p-phenylenediamine, 4-amino-3-methylphenol, 2-aminomethyl-4-aminophenol, 4-amino-2-((diethylamino)-methyl)-phenol, bis-(2-hydroxy-5-aminophenyl)-methane, 2,4,5,6-tetraaminopyrimidine, 2-hydroxy-4,5,6-triaminopyrimidine and 4-hydroxy-2,5,6-triaminopyrimidine and
• manganese cations with oxidation numbers above 2 in the form of their salts soluble in the preparation applied and/or their complexes.

2. A preparation as claimed in claim 1, **characterized in that** the primary intermediate is selected from a group consisting of p-phenylenediamine, p-toluylenediamine, p-aminophenol, 1-(2'-hydroxyethyl)-2,5-diaminobenzene, N,N-bis-(2-hydroxyethyl)-p-phenylenediamine, 4-amino-3-methylphenol, 2-aminomethyl-4-aminophenol, 2,4,5,6-tetraaminopyrimidine, 2-hydroxy-4,5,6-triaminopyrimidine and 4-hydroxy-2,5,6-triaminopyrimidine.

3. A preparation as claimed in claim 1 or 2, **characterized in that** the manganese cations present have oxidation numbers of 3, 4 and/or 5.

4. A preparation as claimed in any of claims 1 to 3, **characterized in that** the manganese cations are used in the form of the dihydrate of manganese(III) triacetate.

5. A preparation as claimed in any of claims 1 to 4, **characterized in that** the manganese cations are fixed to a carrier.

6. A preparation as claimed in any of claims 1 to 5, **characterized in that** it contains at least one secondary intermediate.

7. A preparation as claimed in any of claims 1 to 6, **characterized in that** it contains at least one substantive dye.

8. A preparation as claimed in any of claims 1 to 7, **characterized in that** it is free from hydrogen peroxide.

9. The use of the preparation claimed in claims 1 to 8 for oxidatively colouring keratinous fibres,

## Revendications

1. Agent pour teindre des fibres de kératine, en particulier les cheveux humains,c aractérisé en ce qu'il contient
• au moins un composant révélateur, choisi dans le groupe formé par la p-phénylènediamine, la p-toluytènediamine, le p-aminophénol, le 1-(2'-hydroxyéthyl)-2,5-diaminobenzène, la N,N-bis(2-hydroxyéthyl)-p-phénylènediamine, le 4-amino-3-méthylphénol, le 2-aminométhyl-4-aminophénol, le 4-amino-2-((diéthylamino)-méthyl)-phénol, le bis-(2-hydroxy-5-amino-phényl)méthane, la 2,4,5,6,-tétraaminopyrimidine, la 2-hydroxy-4,5,6-triaminopyrimidine et la 4-hydroxy-2,5,6-triaminopyrimidine, ainsi que
• des cations de manganèse dans des états d'oxydation supérieurs à 2 sous forme de leurs sels solubles dans la préparation d'application et/ou leurs complexes.

2. Agent selon la revendication 1, **caractérisé en ce que** le composant révélateur est choisi dans un groupe qui est formé par la p-phénylènediamine, la p-toluylènediamine, le p-aminophénol, le 1-(2'-hydroxyéthyl)-2,5-diaminobenzène, la N,N-bis(2-hydroxyéthyl)-p-phénylènediamine, le 4-amino-3-méthylphénol, le 2-aminométhyl-4-aminophénol, la 2,4,5,6-tétraamino-pyrimidine, la 2-hydroxy-4,5,6-triaminopyrimidine et la 4-hydroxy-2,5,6-triaminopyrimidine.

3. Agent selon la revendication 1 ou 2, **caractérisé en ce que**, les cations de manganèse se trouventd ans les états d'oxydation 3, 4 et/ou 5.

4. Agent selon l'une quelconque des revendications 1 à 3, **caractérisé en ce que** les cations de manganèse sont mis en oeuvre sous forme du triacétate de manganèse(III) dihydraté.

5. Agent selon l'une quelconque des revendications 1 à 4, **caractérisé en ce que** les cations de manganèse sont fixés sur un support.

6. Agent selon l'une quelconque des revendications 1 à 5, **caractérisé en ce qu'**il contienta u moins un composantde couplage.

7. Agent selon l'une quelconque des revendications 1 à 6, **caractérisé en ce qu'**il contientau moins un colorant montantdirectement sur les fibres.

8. Agent selon l'une quelconque des revendications 1 à 7, **caractérisé en ce qu'**il estexempt de peroxyde d'hydrogène.

9. Utilisation d'un des agents des revendications 1 à 8, pour la teinture par oxydation de fibres de kératine.
